# EUROPEAN PATENT SPECIFICATION

(11) **EP 3 180 429 B1**
(45) Date of publication and mention of the grant of the patent: **04.03.2020**
(21) Application number: 15734676.8
(22) Date of filing: 07.07.2015
(51) Int. Cl.: C12N 9/96, C12N 11/08, C11D 3/386

(54) **DETERGENTS AND COMPOSITIONS WITH ENZYMATIC POLYMER PARTICLES**
WASCHMITTEL UND ZUSAMMENSETZUNGEN MIT ENZYMATISCHEN POLYMERTEILCHEN
DÉTERGENTS ET COMPOSITIONS AVEC DES PARTICULES DE POLYMÈRE ENZYMATIQUE

(30) Priority: 11.08.2014 EP 14180570
(43) Date of publication of application: 21.06.2017
(73) Proprietor: Novozymes A/S, 2880 Bagsvaerd (DK)
(72) Inventor: RASMUSSEN, Tue, DK-2880 Bagsvaerd (DK); LARSON, Katarina, DK-2880 Bagsvaerd (DK); ANDERSEN, Kim, Bruno, DK-2880 Bagsvaerd (DK); NOERBY, Martin, DK-2880 Bagsvaerd (DK); FOVERSKOV, Morten, DK-2880 Bagsvaerd (DK); SIMONSEN, Ole, DK-2880 Bagsvaerd (DK); HAY, Robert, Neil, Deeside Flintshire CH5 2NT (GB); DUNCALF, David, John, Deeside Flintshire CH5 2NT (GB); YOUNG, Brian, David, Deeside Flintshire CH5 2NT (GB); PEARS, David, Alan, Deeside Flintshire CH5 2NT (GB)
(74) Representative: NZ EPO Representatives
(86) International application number: PCT/EP2015/065500
(87) International publication number: WO 2016/023685

(56) References cited:
- WO-A1-2009/103576
- WO-A1-2010/119022
- WO-A1-2013/103528
- WO-A2-2008/084093
- US-A- 4 090 973
- FRANCESCA CAVALIERI ET AL: "Chaperone-like activity of nanoparticles of hydrophobized poly(vinyl alcohol)", SOFT MATTER, vol. 3, no. 6, 1 January 2007 (2007-01-01) , page 718, XP055142906, ISSN: 1744-683X, DOI: 10.1039/b618779j
- BATTISTEL E ET AL: "Effect of polyvinylalcohols on the thermostability of lipase from", APPLIED BIOCHEMISTRY AND BIOTECHNOLOGY ; PART A: ENZYME ENGINEERING AND BIOTECHNOLOGY, HUMANA PRESS INC, NEW YORK, vol. 50, no. 2, 1 February 1995 (1995-02-01), pages 161-173, XP035175814, ISSN: 1559-0291, DOI: 10.1007/BF02783452
- KATYA CARBONE ET AL: "Crosslinked poly(vinyl alcohol) supports for the immobilization of a lipolytic enzyme", JOURNAL OF APPLIED POLYMER SCIENCE, vol. 74, no. 8, 21 November 1999 (1999-11-21), pages 1881-1889, XP055145437, ISSN: 0021-8995, DOI: 10.1002/(SICI)1097-4628(19991121)74:8<1881 ::AID-APP1>3.0.CO;2-5
- Martin Chaplin: "Preparation of enzymes for sale", Enzyme Technology , 6 August 2014 (2014-08-06), XP002730867, Retrieved from the Internet: URL:http://www1.lsbu.ac.uk/water/enztech/s ale.html [retrieved on 2014-09-26]

## Description

### Reference to a Sequence Listing

This application contains a Sequence Listing in computer readable form. The computer readable form is incorporated herein by reference.

### FIELD OF THE INVENTION

The present invention relates to hydrophobic modified polyvinyl alcohol polymer particles containing enzymes, and liquid laundry detergents and other compositions comprising said polymer particles.

### BACKGROUND

The stability of enzymes is known to be influenced by the surrounding environment upon storage, as chemical or physical factors may decrease the stability of the enzyme. In particular, the stability of enzymes in liquid formulations comprising protein hostile compounds, such as liquid detergents, is problematic and it is difficult to keep the enzymes stable in such liquid formulations. A particular problem associated with liquid detergents is that they usually contain proteolytic enzymes which digest proteins, thus other enzymes present in the liquid detergent might be inactivated by present proteases wherein both proteolysis and autoproteolysis might occur.

To use particles comprising a mixture of polymer and enzyme in liquid formulations instead of usual liquid enzyme products may have several advantages; it is possible to keep enzyme hostile compounds away from the enzyme until the activity of the enzyme is needed and it is possible to avoid the enzyme to be in direct contact with compounds in the liquid which activates the enzyme. However, the liquid formulations may become turbid after addition of enzyme containing polymer particles, due to the light scattering of the relatively large particles. It may also be of importance that the particles do not or only slightly change appearance of the liquid formulation after addition and that they have a decreased tendency to sediment. It may furthermore be of importance that the enzyme is released at the right time, e.g. for a liquid detergent that the enzyme is released upon contact with the wash water.

Therefore it is an objective of the present invention to provide compositions for effectively stabilizing enzymes in liquid formulations comprising protein hostile compounds, such as liquid detergents. It is desirable that the compositions can be easily incorporated into liquid formulations, in particular into liquid detergent compositions. Moreover, the compositions should be easily contrivable.

It has surprisingly been found that these and further objectives are solved by compositions in the form of enzyme containing particles, which comprises at least one polymer P, which is a hydrophobic modified polyvinyl alcohol.

Previous disclosures of using polymers in enzyme containing particles include WO 2008/084093 and WO 2010/003934. However, the specific hydrophobic modified polyvinyl alcohols of the present invention are different, and have different properties, compared to those previously disclosed.

WO 2010/119022 discloses polymer particles comprising polyvinyl alcohol having one or more hydrocarbyl group substituents of from C4 to C22 carbon chain length.

Cavalieri et al., Soft Matter (2007), 3(6), 718, discloses nanoparticles made from polyvinyl alcohol randomly grafted with hydrophobic methacryloyl groups.

Battistel et al., Appl Biochem Biotechnol (1995), 50(2), 161-173, discloses stabilization against thermal inactivation of a lipase from *Candida rugosa,* in the presence of polyvinylalcohols.

US 4,090,973 discloses a liquid detergent comprising a protease or amylase encapsulated with a solid alkoxylated nonionic surfactant.

WO 2013/103528 discloses polymers containing an acetoacetate moiety.

Carbone et al., J Appl Poly Sci (1999), 74(8), 1881-1889, discloses immobilization of a lipolytic enzyme using certain esters of crosslinked polyvinyl alcohol.

WO 2009/103576 discloses particles comprising a benefit agent, and a water soluble polymeric film-forming material modified with a first charged derivatising group(s).

M.Chaplin, Enzyme Technology (2014), http://www1.lsbu.ac.uk/water/enztech/sale.html discloses methods for stabilizing enzyme preparations for sale.

WO 2008/084093 discloses particles comprising an enzyme and a polymer, where the polymer is soluble in an aqueous solution having an ionic strength of 0 mol/kg and insoluble in an aqueous solution having an ionic strength of more than 1 mol/kg.

### SUMMARY OF THE INVENTION

In a first aspect, the present invention provides enzyme compositions in the form of enzyme containing particles, wherein the particles comprise
i) at least one enzyme, and
ii) at least one polymer P,
where the polymer P is a hydrophobically modified polyvinyl alcohol, and the hydrophobic modifying groups are a mixture of keto-ester and butyryl groups such that the total degree of substitution (DS) is between about 3% and 20%.

### DETAILED DESCRIPTION

The enzyme composition of the present invention has several advantages. The particles comprising a mixture of polymer P and enzyme improve storage stability of the enzyme(s) in liquid formulations such as detergents. The enzyme containing polymer particles can be easily produced from liquid enzyme preparations, and furthermore the polymer P is commercially available or can be easily produced. If smaller sized particles are used they are practically invisible in the formulation and do not sediment.

As the enzyme is present in the particles, the enzyme is not in direct contact with the environment and enzyme sensitive compounds in the surrounding environment such as the components of a liquid detergent are not in direct contact with the enzyme. Thus, the enzyme containing particles of the composition protect enzyme sensitive components in liquid detergents from the enzyme, *e.g*., ester based components like perfume or hydrogenated castor oil are protected from lipases, cellulose based materials are protected from cellulases, starch based components like dextrin or cyclodextrin are protected from amylases, protein/peptide based components are protected from proteases, etc.

The enzyme is rapidly released into the media where it is supposed to work. With regard to detergents it is important that the enzyme is released when the detergent is diluted by water during the wash process. This is ensured by the properties of the polymer P, which functions as a release system. Thus, the enzyme composition is suitable for incorporation into detergent compositions, in particular liquid detergent compositions. Therefore the invention also relates to detergent compositions, in particular liquid detergent compositions.

### Enzymes

The enzyme containing particles of the composition of the invention comprise at least one enzyme such as protease, lipase, cutinase, an amylase, carbohydrase, cellulase, pectinase, mannanase, arabinase, galactanase, xylanase, DNAse, perhydrolase, oxidase, e.g., a laccase, and/or peroxidase. Preferably, the enzyme is a detergent enzyme.

The enzyme may be a naturally occurring enzyme of bacterial or fungal origin, or it may be a variant derived from one or more naturally occurring enzymes by gene shuffling and/or by substituting, deleting or inserting one or more amino acids. Chemically modified or protein engineered mutants are included.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, Carezyme™, and Celluclean™ (Novozymes A/S), Clazinase™, and Puradax HA™ (Genencor International Inc.), and KAC-500(B)™ (Kao Corporation).

Proteases: Suitable proteases include those of bacterial, fungal, plant, viral or animal origin, e.g., vegetable or microbial origin. Microbial origin is preferred. Chemically modified or protein engineered mutants are included. It may be an alkaline protease, such as a serine protease or a metalloprotease. A serine protease may for example be of the S1 family, such as trypsin, or the S8 family such as subtilisin. A metalloproteases protease may for example be a thermolysin from, e.g., family M4 or other metalloprotease, such as those from M5, M7 or M8 families.

The term "subtilases" refers to a sub-group of serine protease according to Siezen et al., Protein Engng. 4 (1991) 719-737 and Siezen et al. Protein Science 6 (1997) 501-523. Serine proteases are a subgroup of proteases characterized by having a serine in the active site, which forms a covalent adduct with the substrate. The subtilases may be divided into 6 sub-divisions, *i.e.,* the Subtilisin family, the Thermitase family, the Proteinase K family, the Lantibiotic peptidase family, the Kexin family and the Pyrolysin family.

Examples of subtilases are those derived from *Bacillus* such as *Bacillus lentus, B. alkalophilus, B. subtilis, B. amyloliquefaciens, Bacillus pumilus* and *Bacillus gibsonii* described in; US7262042 and WO09/021867, and subtilisin lentus, subtilisin Novo, subtilisin Carlsberg, Bacillus licheniformis, subtilisin BPN', subtilisin 309, subtilisin 147 and subtilisin 168 described in WO89/06279 and protease PD138 described in WO93/18140. Other useful proteases may be those described in WO92/175177, WO01/016285, WO02/026024 and WO02/016547. Examples of trypsin-like proteases are trypsin (e.g., of porcine or bovine origin) and the *Fusarium* protease described in WO89/06270, WO94/25583 and WO05/040372, and the chymotrypsin proteases derived from *Cellumonas* described in WO05/052161 and WO05/052146.

A further preferred protease is the alkaline protease from *Bacillus lentus* DSM 5483, as described for example in WO95/23221, and variants thereof which are described in WO92/21760, WO95/23221, EP1921147 and EP1921148.

Examples of metalloproteases are the neutral metalloprotease as described in WO07/044993 (Genencor Int.) such as those derived from *Bacillus amyloliquefaciens.*

Examples of useful proteases are the variants described in: WO92/19729, WO96/034946, WO98/20115, WO98/20116, WO99/011768, WO01/44452, WO03/006602, WO04/03186, WO04/041979, WO07/006305, WO11/036263, WO11/036264, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Suitable commercially available protease enzymes include those sold under the trade names Alcalase®, Duralase^{Tm}, Durazym^{Tm}, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Neutrase®, Everlase® and Esperase® (Novozymes A/S), those sold under the tradename Maxatase®, Maxacal®, Maxapem®, Purafect®, Purafect Prime@, Preferenz^{Tm}, Purafect MA®, Purafect Ox®, Purafect OxP®, Puramax®, Properase®, Effectenz^{Tm}, FN2®, FN3®, FN4®, Excellase®, Opticlean®, Optimase®, and Excellenz P1000 (Danisco/DuPont), Axapem™ (Gist-Brocases N.V.), BLAP (sequence shown in Figure 29 of US5352604) and variants hereof (Henkel AG) and KAP (*Bacillus alkalophilus* subtilisin) from Kao.

Lipases and Cutinases: Suitable lipases and cutinases include those of bacterial or fungal origin. Chemically modified or protein engineered mutant enzymes are included. Examples include lipase from *Thermomyces, e.g.,* from *T. lanuginosus* (previously named *Humicola lanuginosa)* as described in EP258068 and EP305216, cutinase from *Humicola, e.g., H. insolens* (WO96/13580), lipase from strains of *Pseudomonas* (some of these now renamed to *Burkholderia), e.g., P. alcaligenes* or *P. pseudoalcaligenes* (EP218272), *P. cepacia* (EP331376), *P. sp.* strain SD705 (WO95/06720 & WO96/27002), *P. wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S*. *pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include include Lipolase™, Lipex™; Lipolex™ and Lipoclean™ (Novozymes A/S), Lumafast (originally from Genencor) and Lipomax (originally from Gist-Brocades).

Still other examples are lipases sometimes referred to as acyltransferases or perhydrolases, *e.g.,* acyltransferases with homology to *Candida antarctica* lipase A (WO10/111143), acyltransferase from *Mycobacterium smegmatis* (WO05/56782), perhydrolases from the CE 7 family (WO09/67279), and variants of the *M*. *smegmatis* perhydrolase in particular the S54V variant used in the commercial product Gentle Power Bleach from Huntsman Textile Effects Pte Ltd (WO10/100028).

In an embodiment of the invention, the amino acid sequence of the lipase has at least 70% sequence identity, preferably at least 75%, more preferably at least 80%, more preferably at least 85%, more preferably at least 90%, more preferably at least 95%, 96%, 97%, 98%, 99%, and most preferably 100% sequence identity to the amino acid sequence of SEQ ID NO: 1. In another embodiment, the number of amino acid substitutions, deletions and/or insertions introduced into the amino acid sequence of SEQ ID NO: 1 is up to 10, e.g., 1, 2, 3, 4, 5, 6, 7, 8, 9, or 10; or up to 5, e.g., 1, 2, 3, 4, or 5.

Amylases: Suitable amylases are alpha-amylases or glucoamylases and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 3 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193. Other amylases which are suitable are hybrid alpha-amylase comprising residues 1-33 of the alpha-amylase derived from *B*. *amyloliquefaciens* shown in SEQ ID NO: 6 of WO 2006/066594 and residues 36-483 of the *B*. *licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from *B*. *amyloliquefaciens* shown in SEQ ID NO: 6 of WO 2006/066594 and residues 36-483 of SEQ ID NO: 4 are those having the substitutions:
M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Duramyl™, Termamyl™, Fungamyl™, Stainzyme™, Stainzyme Plus™, Natalase™, Liquozyme X and BAN™ (from Novozymes A/S), and Rapidase™, Purastar™/Effectenz™, Powerase and Preferenz S100 (from Genencor International Inc./DuPont).

Lyase: The lyase may be a pectate lyase of bacterial or fungal origin. Chemically or genetically modified mutants are included. In a preferred embodiment the pectate lyase is derived from *Bacillus,* particularly *Bacillus substilis, B. licherniformis* or *B*. *agaradhaerens,* or a variant derived of any of these, e.g. as described in US 6,124,127, WO 1999/027083, WO 1999/027084, WO 2002/006442, WO 2002/092741, WO 2003/095638, Commercially available pectate lyases include XPect; Pectawash and Pectaway (Novozymes A/S).

Mannanase: Suitable mannanases include those of bacterial or fungal origin. Chemically or genetically modified mutants are included. The mannanase may be an alkaline mannanase of Family 5 or 26. It may be a wild-type from *Bacillus* or *Humicola,* particularly B. *agaradhaerens, B. licheniformis, B. halodurans, B. clausii,* or *H. insolens.* Suitable mannanases are described in WO 1999/064619. A commercially available mannanase is Mannaway (Novozymes A/S).

Deoxyribonuclease (DNase): Suitable deoxyribonucleases (DNases) are any enzyme that catalyzes the hydrolytic cleavage of phosphodiester linkages in the DNA backbone, thus degrading DNA. According to the invention, a DNase which is obtainable from a bacterium is preferred; in particular a DNase which is obtainable from a *Bacillus* is preferred; in particular a DNase which is obtainable from *Bacillus subtilis* or *Bacillus licheniformis* is preferred. Examples of such DNases are described in patent application WO 2011/098579 or in PCT/EP2013/075922.

Perhydrolase: Suitable perhydrolases are capable of catalyzing a perhydrolysis reaction that results in the production of a peracid from a carboxylic acid ester (acyl) substrate in the presence of a source of peroxygen (*e.g*., hydrogen peroxide). While many enzymes perform this reaction at low levels, perhydrolases exhibit a high perhydrolysis:hydrolysis ratio, often greater than 1. Suitable perhydrolases may be of plant, bacterial or fungal origin. Chemically modified or protein engineered mutants are included.

Examples of useful perhydrolases include naturally occurring *Mycobacterium* perhydrolase enzymes, or variants thereof. An exemplary enzyme is derived from *Mycobacterium smegmatis.* Such enzyme, its enzymatic properties, its structure, and variants thereof, are described in WO 2005/056782, WO 2008/063400, US 2008/145353, and US2007167344.

Peroxidases/Oxidases: Suitable peroxidases include those comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C*. *cinerea* (EP 179,486), and variants thereof as those described in WO 93/24618, WO 95/10602, and WO 98/15257.

A peroxidase according to the invention also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase of the invention is a chloroperoxidase. Preferably, the haloperoxidase is a vanadium haloperoxidase, *i.e.,* a vanadate-containing haloperoxidase. In a preferred method of the present invention the vanadate-containing haloperoxidase is combined with a source of chloride ion.

Haloperoxidases have been isolated from many different fungi, in particular from the fungus group dematiaceous hyphomycetes, such as *Caldariomyces, e.g., C. fumago, Alternaria, Curvularia, e.g., C. verruculosa* and *C*. *inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as *C*. *inaequalis* CBS 102.42 as described in WO 95/27046; or *C*. *verruculosa* CBS 147.63 or *C*. *verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase according to the invention include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C*. *cinerea, C. comatus, C. friesii,* and *C*. *plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase derived from *Coprinopsis* or *Myceliophthora* is preferred; in particular a laccase derived from *Coprinopsis cinerea,* as disclosed in WO 97/08325; or from *Myceliophthora thermophila,* as disclosed in WO 95/33836.

Amino acid changes in SEQ ID NO: 1, as referenced above, may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for enzyme activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g*., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

The relatedness between two amino acid sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

### Polymers

The enzyme containing particles of the composition of the invention comprise at least one polymer P, where the polymer P is a hydrophobically modified polyvinyl alcohol, and the hydrophobic modifying groups are a mixture of keto-ester and butyryl groups such that the total degree of substitution (DS) is between about 3% and 20%.

In general, preferred PVOH or PVOH based polymers which are suitable in this application have high levels of hydrolysis within the range 60-99%. Most preferred hydrolysis levels are between 88-99% as these polymers have suitable water solubility characteristics. PVOH or PVOH based polymers which are preferred in this application have average molecular weights ranging from 1000 to 200000 which provide for aqueous solutions which are easily handled. Most preferred molecular weights are between 20000 and 80000. The description 'Polyvinyl alcohol -PVOH' will include copolymers containing, for example, vinyl acetate comonomers at varying degrees according to the degree of hydrolysis of the PVOH and ethylene.

Preferred modified PVOH materials may be produced via the post reaction of a suitable acetoacetylation agent directly with the 'alcohol functionality of the parent PVOH based polymer or copolymer. Suitable derivatising agents include; diketene, diketene acetone adduct and tert-butyl acetoacetate.

Most preferred modified PVOH polymers may be produced via the reaction of a C4 aldehyde directly with the alcohol functionality of the acetoacetylised PVOH.

The degree of modification of the PVOH based polymer may be between 3% and 20%, by this it is meant that the 'OH' portion of the PVOH has been replaced by the given molar percentage. The person skilled in the art will appreciate that, for example, in the case of the reaction of an aldehyde with 'PVOH' for each molar quantity of aldehyde two molar quantities of 'OH' are substituted via the acetalation reaction. Hence a 10% modified PVOH will have been reacted with 5% of a suitable aldehyde, and, of course the degree of hydrolysis of the PVOH will dictate the maximum level of substitution possible.

Modified PVOH is described in WO 2004/031271 and WO2009/103576. WO 2004/031271 describes the synthesis and process by which suitable modifications to PVOH may be made in order to produce a modified PVOH film which is resistant to dissolution in concentrated surfactant solution but which dissolves quickly when the surfactant solution is diluted sufficiently. WO2009/103576 also describes how multiple modifications may be made to modify PVOH and further describes how particles may be produced which are coated in this modified PVOH.

In one preferred embodiment of the invention, the modified PVOH is present in an amount of from about 0.1 to about 99% based on the weight of the total coating.

### Enzyme containing particles

The enzyme containing particles of the composition of the invention comprise at least one polymer P, as defined above, and one or more of the enzymes described above.

The enzyme containing particles preferably comprise the at least one enzyme and the at least one polymer P in a weight ratio of enzyme to polymer P from 1:50 to 10:1, in particular from 1:40 to 5:1, more preferably from 1:30 to 2:1. Preferably the total amount of the at least one enzyme and the at least one polymer P is at last 30 %, in particular at least 40 % and more preferably at least 50 % of the enzyme containing particles.

The composition of the invention may additionally contain further components which are generally useful in particulate enzyme compositions. The amount of these further components will generally not exceed 70 % by weight, in particular not exceed 60 % by weight and more preferably not exceed 50 % by weight.

Additional components, which can be incorporated into the particles include e.g. polysaccharides, waxes, enzyme activators or enhancing agents, fillers, enzyme stabilizing agents, solubilising agents, crosslinking agents, suspension agents, viscosity regulating agents, light spheres, chlorine scavengers, plasticizers, pigments, salts, preservatives and fragrances.

Suitable polysaccharides may be un-modified naturally occurring polysaccharides or modified naturally occurring polysaccharides. Suitable polysaccharides include cellulose, pectin, dextrin and starch. The starches may be soluble or insoluble in water. In a particular embodiment of the present invention the polysaccharide is a starch. In a particular embodiment of the present invention the polysaccharide is an insoluble starch. Naturally occurring starches from a wide variety of plant sources are suitable (either as starches per se, or as the starting point for modified starches), and relevant starches include starch e.g. from: rice, corn, wheat, potato, oat, cassava, sago-palm, yuca, barley, sweet potato, sorghum, yams, rye, millet, buckwheat, arrowroot, taro, tannia, and may for example be in the form of flour. Cassava starch is among preferred starches in the context of the invention; in this connection it may be mentioned that cassava and cassava starch are known under various synonyms, including tapioca, manioc, mandioca and manihot. As employed in the context of the present invention, the term "modified starch" denotes a naturally occurring starch, which has undergone some kind of at least partial chemical modification, enzymatic modification, and/or physical or physicochemical modification, and which - in general - exhibits altered properties relative to the "parent" starch.

A "wax" in the context of the present invention is to be understood as a polymeric material having a melting point between 25 -150 °C, particularly 30 to 100°C more particularly 35 to 85°C most particularly 40 to 75°C. The wax is preferably in a solid state at room temperature, 25°C. The lower limit is preferred to set a reasonable distance between the temperature at which the wax starts to melt to the temperature at which the particles or compositions comprising the particles are usually stored, 20 to 30°C. For some particles, e.g. particles used in the detergent industry, a preferable feature of the wax is that the wax should be water soluble or water dispersible, particularly in neutral and alkaline solution, so that when the coated particles of the invention is introduced into an aqueous solution, i.e. by diluting it with water, the wax should disintegrate and/or dissolve providing a quick release and dissolution of the active incorporated in the particles to the aqueous solution. Examples of water soluble waxes are poly ethylene glycols (PEG's). Amongst water insoluble waxes, which are dispersible in an aqueous solution are triglycerides and oils. For some particles it is preferable that the coating contains some insoluble waxes e.g. feed particles.

The wax may be any wax, which is chemically synthesized or a wax isolated from a natural source or a derivative thereof. Accordingly, suitable waxes are selected from the following non limiting list of waxes.
- Polyethylene glycols, PEG. Different PEG waxes are commercially available having different molecular sizes, wherein PEG's with low molecular sizes also have low melting points. Examples of suitable PEG's are PEG 1500, PEG 2000, PEG 3000, PEG 4000, PEG 6000, PEG 8000, PEG 9000 etc. e.g. from BASF (Pluriol E series) or from Clariant or from Ineos. Derivatives of Poly ethylene glycols may also be used.
- polypropylene glycols (e.g. polypropylen glycol Pluriol P series from BASF) and polyethylenglycol-polypropylenglycol blockcopolymers. Derivatives of polypropyleneglycols or polyethylenglycol-polypropylenglycol blockcopolymers may also be used.
- Nonionic surfactants which are solid at room temperature such as ethoxylated fatty alcohols having a high level of ethoxy groups such as the Lutensol AT series from BASF, a C₁₆-C₁₈ fatty alcohol having different amounts of ethyleneoxide per molecule, e.g. Lutensol AT11, AT13, AT25, AT50, AT80, where the number indicate the average number of ethyleneoxide groups. Alternatively, polymers of ethyleneoxide, propyleneoxide or copolymers thereof are useful, such as in block polymers, e.g. Pluronic PE 6800 from BASF. Derivatives of ethoxylated fatty alcohols are preferred.
- Waxes isolated from a natural source, such as Carnauba wax (melting point between 80-88°C), Candelilla wax (melting point between 68-70°C) and bees wax. Other natural waxes or derivatives thereof are waxes derived from animals or plants, e.g. of marine origin. Hydrogenated plant oil or animal tallow are likewise suitable. Examples of such waxes are hydrogenated ox tallow, hydrogenated palm oil, hydrogenated cotton seeds and/or hydrogenated soy bean oil, wherein the term "hydrogenated" as used herein is to be construed as saturation of unsaturated carbohydrate chains, e.g. in triglycerides, wherein carbon=carbon double bonds are converted to carbon-carbon single bonds. Hydrogenated palm oil is commercially available e.g. from Hobum Oele und Fette GmbH - Germany or Deutche Cargill GmbH - Germany.
- Fatty acid alcohols, such as the linear long chain fatty acid alcohol NAFOL 1822 (C18, 20, 22) from Condea Chemie GMBH - Germany, having a melting point between 55-60°C. Derivatives of fatty acid alcohols are likewise useful.
- Monoglycerides and/or di-glycerides, such as glyceryl stearate, wherein stearate is a mixture of stearic and palmitic acid, are useful waxes. An example of this is Dimodan PM - from Danisco Ingredients, Denmark.
- Fatty acids, such as hydrogenated linear long chained fatty acids and derivatives of fatty acids.
- Paraffines, i.e. solid hydrocarbons.
- Micro-crystalline wax.

Further suitable waxes can be found in C.M. McTaggart et. al., Int. J. Pharm. 19, 139 (1984) or Flanders et. al., Drug Dev. Ind. Pharm. 13, 1001 (1987).

In a particular embodiment of the present invention the wax of the present invention is a mixture of two or more different waxes. In a particular embodiment of the present invention the wax or waxes is selected from the group consisting of PEG, ethoxylated fatty alcohols, fatty acids, fatty acid alcohols and glycerides. In another particular embodiment of the present invention the waxes are chosen from synthetic waxes. In a more particular embodiment the waxes of the present invention are PEG or nonionic surfactants. In a most particular embodiment of the present invention the wax is PEG.

Suitable fillers are water soluble and/or insoluble inorganic salts such as finely ground alkali sulphate, alkali carbonate and/or alkali chloride, clays such as kaolin (e.g. SPESWHITE®, English China Clay), bentonites, talcs, zeolites, chalk, calcium carbonate and/or silicates. Typical fillers are di-sodium sulphate and calcium-lignosulphonate. Other fillers are silica, gypsum, kaolin, talc, magnesium aluminium silicate and cellulose fibres.

Suitable enzyme stabilizing or -protective agents may fall into several categories and include, e.g. alkaline or neutral materials, reducing agents, antioxidants and/or salts of first transition series metal ions. Each of these may be used in conjunction with other protective agents of the same or different categories. Examples of alkaline protective agents are alkali metal silicates, -carbonates or bicarbonates which provide a chemical scavenging effect by actively neutralizing e.g. oxidants. Examples of reducing protective agents are salts of sulfite, thiosulfite or thiosulfate, while examples of antioxidants are methionine, butylated hydroxytoluene (BHT) or butylated hydroxyanisol (BHA). Most preferred agents are salts of thiosulfates, e.g. sodium thiosulfate. Also, enzyme stabilizers may be borates, borax, formates, di- and tricarboxylic acids and so called reversible enzyme inhibitors such as organic compounds with sulfhydryl groups or alkylated or arylated boric acids.

Suitable cross-linking agents include e.g. enzyme-compatible surfactants, e.g. ethoxylated alcohols, especially ones with 10 to 80 ethoxy groups.

The solubility of the particle may be critical in cases where the coated particle is a component of a detergent formulation. As is known by the person skilled in the art, many agents, through a variety of methods, serve to increase the solubility of formulations, and typical agents known to the art can be found in National Pharmacopeia's.

Light spheres are small particles with low true density. Typically, they are hollow spherical particles with air or gas inside. Such materials are usually prepared by expanding a solid material. These light spheres may be inorganic of nature or organic of nature, such as the PM-series (plastic hollow spheres) available from The PQ Corporation. Light spheres can also be prepared from polysaccharides, such as starch or derivatives thereof. Biodac® is an example of non-hollow lightweight material made from cellulose (waste from papermaking), available from GranTek Inc. These materials may be included in the particles of the invention either alone or as a mixture of different light materials.

Suspension agents, mediators (for boosting bleach action upon dissolution of the particle in e.g. a washing application) and/or solvents may be incorporated in the particle.

Plasticizers useful in particles in the context of the present invention include, for example: polyols such as sugars, sugar alcohols, glycerine, glycerol trimethylol propane, neopentyl glycol, triethanolamine, mono-, di- and triethylene glycol or polyethylene glycols (PEGs) having a molecular weight less than 1000; urea, phthalate esters such as dibutyl or dimethyl phthalate; thiocyanates, non-ionic surfactants such as ethoxylated alcohols and ethoxylated phosphates and water.

Suitable pigments include, but are not limited to, finely divided whiteners, such as titanium dioxide or kaolin, coloured pigments, water soluble colorants, as well as combinations of one or more pigments and water soluble colorants.

Suitable salts which can be incorporated in the particles may be any inorganic salt, e.g. salts of sulfate, sulfite, phosphate, phosphonate, nitrate, chloride or carbonate or salts, or salts of simple organic acids (less than 10 carbon atoms e.g. 6 or less carbon atoms) such as citrate, malonate or acetate. Examples of cations in these salts are alkali or earth alkali metal ions, although the ammonium ion or metal ions of the first transition series, such as sodium, potassium, magnesium, calcium, zinc or aluminium. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular alkali- or earth alkali metal salts of sulfate, sulfite, phosphate, phosphonate, nitrate, chloride or carbonate or salts of simple organic acids such as citrate, malonate or acetate may be used. Specific examples include NaH₂PO₄, Na₂HPO₄, Na₃PO₄, (NH₄)H₂PO₄, K₂HPO₄, KH₂PO₄, Na₂SO₄, K₂SO₄, KHSO₄, ZnSO₄, MgSO₄, CuSO₄, Mg(NO₃)₂, (NH₄)₂SO₄, sodium borate, magnesium acetate and sodium citrate. The salt may also be a hydrated salt, i.e. a crystalline salt hydrate with bound water(s) of crystallization, such as described in WO 99/32595. Examples of hydrated salts include magnesium sulfate heptahydrate (MgSO₄(7H₂O)), zinc sulfate heptahydrate (ZnSO₄(7H₂O)), copper sulfate pentahydrate (CuSO₄(5H₂O)), sodium phosphate dibasic heptahydrate (Na₂HPO₄(7H₂O)), magnesium nitrate hexahydrate (Mg(NO₃)₂(6H₂O)), sodium borate decahydrate, sodium citrate dihydrate and magnesium acetate tetrahydrate.

In the particles of the invention, the polymer and the enzyme and the optional further components will usually present as an intimate mixture, i.e. the distribution of the ingredients within the particles is homogenous or virtually homogenous. However, the particles may also have a core shell structure. The term "core shell structure" means that the distribution of the components within the particles is not homogenous. Rather, at least one component of the particles is predominantly located in the inner region of the particle while at least one other component is predominantly located in the outer region of the particle. In these core shell particles, the enzyme is preferably located in the inner region of the particle. In a particular preferred embodiment of the invention, the distribution of the components within the particles is homogenous or virtually homogenous.

The particle size of the particles in the composition of the invention may vary. Preferably the volume average particle diameter of the enzyme containing particles is from 50 nm to 100 µm, in particular from 100 nm to 80 µm, more preferably from 200 nm to 50 µm, especially from 0.5 to 20 µm. However, the volume average particle diameter may be as small as from 50 to 500 nm.

In some applications it may be desirable to have large particles that are visual by the naked eye, thus the volume average particle diameter of the enzyme containing particles may be from 100 µm to 2000 µm.

Preferably, at least 90% by weight of the particles have a particle diameter of at most 150 µm, in particular at most 100 µm, more preferably at most 70 µm and especially at most 30 µm.

The particle size may be determined by conventional techniques such as light scattering as described e.g. in D. Distler, Wässrige Polymerdispersionen [Aqueos Polymer Dispersions], Wiley-VCH 1999, chapter 4.2.1, p. 40ff, H. Auweter, D. Horn, J. Colloid Interf. Sci. 105 (1985) 399, D. Lilge, D. Horn, Colloid Polym. Sci. 269 (1991) 704 or H. Wiese, D. Horn, J. Chem. Phys. 94 (1991) 6429 or W. Brown, Dynamic Light Scattering Oxford University Press, 1992.

The particles of the present invention may comprise one, two or more additional coating layers. In a particular embodiment of the present invention the particle comprise at least two coating layers.

Additional coatings may be applied to the particle to provide additional characteristics or properties. Thus, for example, an additional coating may achieve one or more of the following effects:
(i) further protection of the active compound in the particle against hostile compounds in the surroundings.
(ii) dissolution at a desired rate upon introduction of the particle into a liquid medium (such as an acid medium);
(iii) provide a better physical strength of the particle.

In a particular embodiment of the present invention an outer layer may be applied as known within microencapsulation technology, e.g. via polycondensation as interfacial polymerization and in situ polymerization, coacervation, gelation and chelation, solvent extraction, evaporation and suspension crosslinking. Different coating techniques are described in "Microspheres, Microcapsules and Liposomes", ed. Reza Arshady, Citus Books Ltd. and in WO 97/24179.

Generally, the enzyme particles may be in the form of a powder or in the form of a dispersion in a liquid medium. Frequently, a powder is prepared in the first step, which is in a second step incorporated into a liquid medium, e.g. a polar liquid medium such as an aqueous liquid or a liquid emulsifier, liquid mixtures of emulsifiers or mixtures thereof, or non-polar liquid medium such as a liquid hydrocarbon or a liquid plant oil or mixtures thereof. In a particular preferred embodiment, the liquid medium is a liquid surfactant or a liquid mixture of surfactants (liquid emulsifiers) or contains at least 80 % by weight, based on the weight of the liquid medium of at least one liquid surfactant or surfactant mixture. Examples of liquid surfactants are non-ionic surfactants like alcohol alkoxylates, in particular. Such dispersions can also contain various additives, e.g. to stabilize against sedimentation. The type of liquid medium is of minor importance and mainly depends of the intended purpose for the enzyme particles. It is, however, also possible to use processes, where the enzyme containing particles are obtained as a liquid dispersion.

The preparation of the compositions according to the invention can be accomplished by customary methods for the preparation of particulate substances whose particles comprise a plurality of components. As a rule, the components of the active substance-containing particles are mixed with one another and then processed by customary methods to give the enzyme particles. Such a process is also subject matter of the present application.

Examples of processes which are suitable in accordance with the invention are coprecipitation and drying methods such as spray drying, fluidized-bed drying, fluidized-bed coating, preparation of Pickering dispersions with subsequent spray drying, and processes which include steps of particle size reduction of larger particles size such as micronization, dry or wet milling.

Preferably, the process for preparing the enzyme particles comprise the steps of preparing a solution of the enzyme and the polymer P, atomizing this solution in a gas or a liquid to make small droplets (atomizing in a gas correspond to a spray drying process, atomizing in a water immiscible liquid gives an emulsion) and drying these droplets to form solid particles. Suitable methods for preparing the enzyme particles include spray drying and emulsion processes.
a) Spray drying process, wherein a liquid enzyme-containing solution is atomized in a spray drying tower to form small droplets which during their way down the drying tower dry to form an enzyme-containing particulate material. Very small particles can be produced this way (Michael S. Showell (editor); Powdered detergents; Surfactant Science Series; 1998; vol. 71; page 140-142; Marcel Dekker).
b) Emulsion process, wherein an aqueous liquid enzyme containing solution is emulsified in a water immiscible liquid e.g. paraffinic oil. To ease the formation of droplets and stabilize the emulsion various emulsifiers and surfactants are used. The water from the droplet can subsequently be removed be distilliation, e.g. azeotropic distillation, or by spray drying the emulsion if the water immiscible liquid is volatile. For emulsions the drying process can be azeotropic distillation as described e.g. in EP 0356239.

The particles of the invention may also be prepared by a size reduction process, wherein preformed larger particles/briquettes or the like are reduced in particle size via milling the larger particles. This can be performed on dry particles (dry milling) or using a dispersion of the particles in a liquid, a so-called slurry (wet milling).

The particles of the invention may also be prepared by a coprecipitation process. Coprecipitation is described for example in WO99/00013, the disclosure of which is herewith referred to.

In accordance with a preferred embodiment of the invention, the preparation of the composition according to the invention is accomplished by a spray-drying method, i.e. by spray drying a liquid composition containing the at least one enzyme and the at least one polymer P. In accordance with a particular preferred embodiment of the invention, the preparation of the composition according to the invention is accomplished by spray drying an aqueous composition containing the at least one enzyme and the at least one polymer P.

To this end, in a first step, the components of the enyzme-containing particles will be mixed with one another, or dissolved, in a suitable solvent or diluent. The resulting suspension or solution will subsequently be subjected to a spray-drying method. Here, the solvent or diluent is removed with the aid of a stream of warm gas, where the components of the active substance particles which are present in the solution or suspension form a finely divided powder which can be obtained in a manner known per se. As an alternative, the components of the particles can be dissolved or dispersed separately and the resulting solutions or dispersions can be subjected to concomitant spray-drying.

In the preparation of the composition according to the invention by a spray-drying method, the components of the particles will, in a first step, be dissolved or suspended in a suitable solvent or diluent. Preferred solvents are those in which all components of the active substance-containing particles dissolve and which do not destroy the enzyme employed. Examples of suitable solvents are:
- water,
- C₁-C₄-alkanols such as methanol, ethanol, propanol, isopropanol, n-butanol, 2-butanol, isobutanol;
- esters of C₁-C₄-aliphatic acids with C₁-C₄-alkanols such as ethyl acetate, butyl acetate, methyl butyrate;
- aliphatic and alicyclic ethers with preferably 4 to 10 C atoms such as tetrahydrofuran, dioxane, diethyl ether, diisopropyl ether, methyl tert-butyl ether;
- halohydrocarbons such as dichloromethane, trichloromethane, dichloroethane;
- cyclic or open-chain carbonates such as ethylene carbonate, propylene carbonate, diethyl carbonate;
- and mixtures of the abovementioned solvents and mixtures of the abovementioned solvents with water.

Preferably an aqueous composition containing the at least one enzyme and the at least one polymer P is subjected to the spray drying. Thus, the solvent is preferably selected from water or a mixture of water and an organic solvent which is miscible with water. Preferably the amount of water in the solvent is at least 50 vol.-%. More preferably water is the only solvent or diluent. Preferably the water or the mixture of water and organic solvent has a pH in the range from pH 6 to pH 9.

In a second step, the solvent is subsequently removed in a suitable spray apparatus with the aid of a stream of warm gas. To this end, the solution(s) or dispersion(s) is/are sprayed into a stream of warm air in a suitable apparatus. Spraying in the solution(s) or dispersion(s) can be effected in cocurrent or in countercurrent with the stream of warm air, preferably in cocurrent, i.e. in the same direction as the stream of warm air.

Suitable apparatuses for spraying in are single- or multi-substance nozzles and atomizer disks.

The temperature of the stream of warm gas, hereinbelow also referred to as drying gas, is typically in the range of from 50 to 200°C, in particular in the range of from 70 to 180°C and specifically in the range of from 100 to 160°C upon entering into the drying apparatus. When the drying gas leaves the drying apparatus, its temperature is typically in the range of from 40 to 120°C and in particular in the range of from 60 to 100°C. Suitable drying gases are, besides air, in particular inert gases such as nitrogen, argon or helium, with nitrogen being preferred. In the case of readily volatile solvents, it is also possible to employ lower temperatures, for example room temperature.

Typically, spray-drying is effected in spray-drying towers which are suitable for this purpose. Here, the solution(s) or dispersion(s) to be dried and the drying gas are typically introduced into the tower at the top. At the bottom of the tower, the dry active substance particles are discharged together with the gas stream and separated from the gas stream in apparatuses which are arranged downstream, such as cyclones. Besides conventional spray-drying, it is also possible to perform an agglomerating spray-drying operation using an internal or external fluidized bed (for example what is known as the FSD technology from Niro), where the particles formed agglomerate to give larger bodies. The primary particle size of the particles formed is, however, preferably in the abovementioned ranges and will in particular not exceed 100 µm and specifically 50 µm.

If appropriate, the particles, in particular when they have a certain tackiness, will be provided with traditional spray-drying adjuvants. These are finely divided solids which are introduced into the spray-drying apparatus together with the solution(s) or dispersion(s) and which ensure that no agglutination or clumping takes place. Suitable finely divided solids are in particular silicas including hydrophobicized silica, alkali metal and alkaline earth metal silicates, alkaline earth metal alumosilicates, highly crosslinked polyvinylpyrrolidone, celluloses, starches, highly crosslinked sodium carboxymethyl starch or crosslinked sodium carboxymethylcellulose. The particle size of these substances is typically below 100 µm (D₉₀ value).

The compositions of the invention can be used in any application, where enzymes are required. The composition of the invention are particularly suitable for incorporation into compositions containing protein hostile substances, e.g. into detergent compositions, in particular into liquid compositions containing protein hostile substances such as liquid detergent compositions.

The present invention also relates to liquid compositions, in particular liquid detergent compositions, containing at least one enzyme containing particle as described herein.

In the detergent compositions of the invention the polymer P protects the enzyme until the detergent is introduced into wash liquor, where the detergent is diluted sufficiently for the particle to dissolve and release the enzyme, so that it is available to act on stains.

The liquid composition of the present composition can be any liquid composition which is suitable to comprise the particles of the invention. The liquid composition may be any composition, but particularly suitable compositions are personal care compositions, cleaning compositions, textile processing compositions e.g. bleaching, pharmaceutical compositions, leather processing compositions, fuel, pulp or paper processing compositions, food and beverage compositions and animal feed compositions.

### Detergent composition

The enzyme containing particles of the invention may be added to and thus become a component of a detergent composition.

The detergent composition may be a solid or a liquid detergent composition. Preferably, the detergent composition is a liquid detergent composition having a physical form, which is not solid (or gas). It may be a pourable liquid, a pourable gel or a non-pourable gel. It may be either isotropic or structured, preferably isotropic. It may be a formulation useful for washing in automatic washing machines or for hand washing.

Liquids, including without limitation, alkanols, amines, diols, ethers and polyols may be included in a liquid detergent. A liquid detergent may contain from 0-30% organic solvent. A liquid detergent may even be substantially non-aqueous, wherein the water content is below 15%, preferably below 10%, more preferably below 6%, more preferably below 4%, more preferably below 2%, and most preferably below 1%.

Detergent ingredients can be separated physically from each other by compartments in water dissolvable pouches. Thereby negative storage interaction between components can be avoided. Different dissolution profiles of each of the compartments can also give rise to delayed dissolution of selected components in the wash solution.

The detergent composition may take the form of a unit dose product. A unit dose product is the packaging of a single dose in a non-reusable container. It is increasingly used in detergents for laundry and dish wash. A detergent unit dose product is the packaging (e.g., in a pouch made from a water soluble film) of the amount of detergent used for a single wash.

Pouches can be of any form, shape and material which is suitable for holding the composition, e.g., without allowing the release of the composition from the pouch prior to water contact. The pouch is made from water soluble film which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably the level of polymer in the film for example PVA is at least about 60%. Preferred average molecular weight will typically be about 20,000 to about 150,000. Films can also be a blend compositions comprising hydrolytically degradable and water soluble polymer blends such as polyactide and polyvinyl alcohol plus plasticizers like glycerol, ethylene glycerol, Propylene glycol, sorbitol and mixtures thereof. The pouches can comprise a solid laundry cleaning composition or part components and/or a liquid cleaning composition or part components separated by the water soluble film. The compartment for liquid components can be different in composition than compartments containing solids (see e.g., US 2009/0011970).

The choice of detergent components may include, for textile care, the consideration of the type of textile to be cleaned, the type and/or degree of soiling, the temperature at which cleaning is to take place, and the formulation of the detergent product. Although components mentioned below are categorized by general header according to a particular functionality, this is not to be construed as a limitation, as a component may comprise additional functionalities as will be appreciated by the skilled artisan.

The choice of additional components is within the skill of the artisan and includes conventional ingredients, including the exemplary non-limiting components set forth below.

### Surfactants

The detergent composition may comprise one or more surfactants, which may be anionic and/or cationic and/or non-ionic and/or semi-polar and/or zwitterionic, or a mixture thereof. In a particular embodiment, the detergent composition includes a mixture of one or more nonionic surfactants and one or more anionic surfactants. The surfactant(s) is typically present at a level of from about 0.1% to 60% by weight, such as about 1% to about 40%, or about 3% to about 20%, or about 3% to about 10%. The surfactant(s) is chosen based on the desired cleaning application, and includes any conventional surfactant(s) known in the art. Any surfactant known in the art for use in detergents may be utilized.

When included therein the detergent will usually contain from about 1% to about 40% by weight, such as from about 5% to about 30%, including from about 5% to about 15%, or from about 20% to about 25% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the detergent will usually contain from about 0.1% to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the detergent will usually contain from about 0.2% to about 40% by weight of a non-ionic surfactant, for example from about 0.5% to about 30%, in particular from about 1% to about 20%, from about 3% to about 10%, such as from about 3% to about 5%, or from about 8% to about 12%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), as well as products available under the trade names SPAN and TWEEN, and combinations thereof.

When included therein the detergent will usually contain from about 0.1% to about 20% by weight of a semipolar surfactant. Non-limiting examples of semipolar surfactants include amine oxides (AO) such as alkyldimethylamineoxide, *N*-(coco alkyl)-*N*,*N*-dimethylamine oxide and N-(tallow-alkyl)-*N,N*-bis(2-hydroxyethyl)amine oxide, fatty acid alkanolamides and ethoxylated fatty acid alkanolamides, and combinations thereof.

When included therein the detergent will usually contain from about 0.1% to about 10% by weight of a zwitterionic surfactant. Non-limiting examples of zwitterionic surfactants include betaine, alkyldimethylbetaine, sulfobetaine, and combinations thereof.

### Hydrotropes

A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however the molecular structure of hydrotropes generally do not favor spontaneous self-aggregation, see for example review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in detergent compositions allow for example more concentrated formulations of surfactants (as in the process of compacting liquid detergents by removing water) without inducing undesired phenomena such as phase separation or high viscosity.

The detergent may contain 0-5% by weight, such as about 0.5 to about 5%, or about 3% to about 5%, of a hydrotrope. Any hydrotrope known in the art for use in detergents may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

### Builders and Co-Builders

The detergent composition may contain about 0-65% by weight, such as about 5% to about 50% of a detergent builder or co-builder, or a mixture thereof. In a dish wash detergent, the level of builder is typically 40-65%, particularly 50-65%. The builder and/or co-builder may particularly be a chelating agent that forms water-soluble complexes with Ca and Mg ions. Any builder and/or co-builder known in the art for use in laundry detergents may be utilized. Non-limiting examples of builders include citrates, zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The detergent composition may also contain 0-50% by weight, such as about 5% to about 30%, of a detergent co-builder, or a mixture thereof. The detergent composition may include include a co-builder alone, or in combination with a builder, for example a citrate builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N*,*N'-*disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N-*methyliminodiacetic acid (MIDA), α-alanine-*N*, *N*-diacetic acid (a-ALDA), serine-*N*, *N*-diacetic acid (SEDA), isoserine-*N*, *N*-diacetic acid (ISDA), phenylalanine-*N*, *N*-diacetic acid (PHDA), anthranilic acid-*N*, *N*-diacetic acid (ANDA), sulfanilic acid-*N*, *N*-diacetic acid (SLDA), taurine-*N*, *N*-diacetic acid (TUDA) and sulfomethyl-*N*, *N*-diacetic acid (SMDA), *N*-(2-hydroxyethyl)-ethylidenediamine-*N*, *N'*, *N'*-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, *e.g*., WO 09/102854, US 5977053.

### Polymers

The detergent may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575 and US 5,955,415. Salts of the above-mentioned polymers are also contemplated.

### Fabric hueing agents

The detergent compositions of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO 2005/03274, WO 2005/03275, WO 2005/03276 and EP 1876226. The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, *e.g*., WO 2007/087257 and WO 2007/087243.

### (Additional) Enzymes

Enzyme(s) which may be comprised in the detergent composition include one or more enzymes such as protease, lipase, cutinase, an amylase, carbohydrase, cellulase, pectinase, mannanase, arabinase, galactanase, xylanase, DNase, perhydrolase, oxidase, *e.g*., laccase, and/or peroxidase. Examples of these enzymes are the same as those included as enzyme particles in the water-soluble film, as described above in the "Enzymes" section.

Such enzyme(s) may be stabilized using conventional stabilizing agents, *e.g*., a polyol such as propylene glycol or glycerol, a sugar or sugar alcohol, lactic acid, boric acid, or a boric acid derivative, *e.g*., an aromatic borate ester, or a phenyl boronic acid derivative such as 4-formyl-phenyl-boronic acid, and the composition may be formulated as described in, for example, WO 92/19709 and WO 92/19708. Other stabilizers and inhibitors as known in the art can be added (see below).

The detergent enzyme(s) may be included in a detergent composition by adding separate additives containing one or more enzymes, or by adding a combined additive comprising all of these enzymes. A detergent additive of the invention, *i.e.,* a separate additive or a combined additive, can be formulated, for example, as a liquid, slurry, or even a granulate, etc.

### Protease Inhibitors

The detergent composition, or the enzyme containing particles of the invention, may include a protease inhibitor, which is a reversible inhibitor of protease activity, *e.g*., serine protease activity. Preferably, the protease inhibitor is a (reversible) subtilisin protease inhibitor. In particular, the protease inhibitor may be a peptide aldehyde, boric acid, or a boronic acid; or a derivative of any of these.

The protease inhibitor may have an inhibition constant to a serine protease, Kᵢ (mol/L) of from 1E-12 to 1E-03; more preferred from 1E-11 to 1E-04; even more preferred from 1E-10 to 1E-05; even more preferred from 1E-10 to 1E-06; and most preferred from 1E-09 to 1E-07.

The protease inhibitor may be boronic acid or a derivative thereof; preferably, phenylboronic acid or a derivative thereof.

In an embodiment of the invention, the phenyl boronic acid derivative is of the following formula: 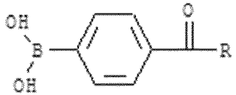 wherein R is selected from the group consisting of hydrogen, hydroxy, C₁-C₆ alkyl, substituted C₁-C₆ alkyl, C₁-C₆ alkenyl and substituted C₁-C₆ alkenyl. Preferably, R is hydrogen, CH₃, CH₃CH₂ or CH₃CH₂CH₂.

In a preferred embodiment, the protease inhibitor (phenyl boronic acid derivative) is 4-formyl-phenyl-boronic acid (4-FPBA).

In another particular embodiment, the protease inhibitor is selected from the group consisting of:
thiophene-2 boronic acid, thiophene-3 boronic acid, acetamidophenyl boronic acid, benzofuran-2 boronic acid, naphtalene-1 boronic acid, naphtalene-2 boronic acid, 2-FPBA, 3-FBPA, 4-FPBA, 1-thianthrene boronic acid, 4-dibenzofuran boronic acid, 5-methylthiophene-2 boronic, acid, thionaphtrene boronic acid, furan-2 boronic acid, furan-3 boronic acid, 4,4 biphenyl-diborinic acid, 6-hydroxy-2-naphtalene, 4-(methylthio) phenyl boronic acid, 4 (trimethylsilyl)phenyl boronic acid, 3-bromothiophene boronic acid, 4-methylthiophene boronic acid, 2-naphtyl boronic acid, 5-bromothiphene boronic acid, 5-chlorothiophene boronic acid, dimethylthiophene boronic acid, 2-bromophenyl boronic acid, 3-chlorophenyl boronic acid, 3-methoxy-2-thiophene, p-methyl-phenylethyl boronic acid, 2-thianthrene boronic acid, dibenzothiophene boronic acid, 4-carboxyphenyl boronic acid, 9-anthryl boronic acid, 3,5 dichlorophenyl boronic, acid, diphenyl boronic acidanhydride, o-chlorophenyl boronic acid, p-chlorophenyl boronic acid, m-bromophenyl boronic acid, p-bromophenyl boronic acid, p-flourophenyl boronic acid, p-tolyl boronic acid, o-tolyl boronic acid, octyl boronic acid, 1,3,5 trimethylphenyl boronic acid, 3-chloro-4-flourophenyl boronic acid, 3-aminophenyl boronic acid, 3,5-bis-(triflouromethyl) phenyl boronic acid, 2,4 dichlorophenyl boronic acid, 4-methoxyphenyl boronic acid.

Further boronic acid derivatives suitable as protease inhibitors in the detergent composition are described in US 4,963,655, US 5,159,060, WO 95/12655, WO 95/29223, WO 92/19707, WO 94/04653, WO 94/04654, US 5442100, US 5488157 and US 5472628.

The protease inhibitor may also be a peptide aldehyde having the formula X-B¹-B⁰-H, wherein the groups have the following meaning:
a) H is hydrogen;
b) B⁰ is a single amino acid residue with L- or D-configuration and with the formula: NH-CHR'-CO;
c) B¹ is a single amino acid residue; and
d) X consists of one or more amino acid residues (preferably one or two), optionally comprising an N-terminal protection group.

NH-CHR'-CO (B⁰) is an L or D-amino acid residue, where R' may be an aliphatic or aromatic side chain, *e.g*., aralkyl, such as benzyl, where R' may be optionally substituted. More particularly, the B⁰ residue may be bulky, neutral, polar, hydrophobic and/or aromatic. Examples are the D- or L-form of Tyr (*p*-tyrosine), *m*-tyrosine, 3,4-dihydroxyphenylalanine, Phe, Val, Met, norvaline (Nva), Leu, Ile or norleucine (Nle).

In the above formula, X-B¹-B⁰-H, the B¹ residue may particularly be small, aliphatic, hydrophobic and/or neutral. Examples are alanine (Ala), cysteine (Cys), glycine (Gly), proline (Pro), serine (Ser), threonine (Thr), valine (Val), norvaline (Nva) and norleucine (Nle), particularly alanine, glycine, or valine.

X may in particular be one or two amino acid residues with an optional N-terminal protection group (i.e. the compound is a tri- or tetrapeptide aldehyde with or without a protection group). Thus, X may be B², B³-B², Z-B², or Z-B³-B² where B³ and B² each represents one amino acid residue, and Z is an N-terminal protection group. The B² residue may in particular be small, aliphatic and/or neutral, e.g., Ala, Gly, Thr, Arg, Leu, Phe or Val. The B³ residue may in particular be bulky, hydrophobic, neutral and/or aromatic, *e.g*., Phe, Tyr, Trp, Phenylglycine, Leu, Val, Nva, Nle or Ile.

The N-terminal protection group Z (if present) may be selected from formyl, acetyl, benzoyl, trifluoroacetyl, fluoromethoxy carbonyl, methoxysuccinyl, aromatic and aliphatic urethane protecting groups, benzyloxycarbonyl (Cbz), t-butyloxycarbonyl, adamantyloxycarbonyl, p-methoxybenzyl carbonyl (MOZ), benzyl (Bn), p-methoxybenzyl (PMB) or p-methoxyphenyl (PMP), methoxycarbonyl (Moc); methoxyacetyl (Mac); methyl carbamate or a methylamino carbonyl/methyl urea group. In the case of a tripeptide aldehyde with a protection group (i.e. X = Z-B²), Z is preferably a small aliphatic group, *e.g*., formyl, acetyl, fluoromethoxy carbonyl, t-butyloxycarbonyl, methoxycarbonyl (Moc); methoxyacetyl (Mac); methyl carbamate or a Methylamino carbonyl/methyl urea group. In the case of a tripeptide aldehyde with a protection group (i.e. X = Z-B³-B²), Z is preferably a bulky aromatic group such as benzoyl, benzyloxycarbonyl, p-methoxybenzyl carbonyl (MOZ), benzyl (Bn), p-methoxybenzyl (PMB) or p-methoxyphenyl (PMP).

Suitable peptide aldehydes are described in WO 94/04651, WO 95/25791, WO 98/13458, WO 98/13459, WO 98/13460, WO 98/13461, WO 98/13461, WO 98/13462, WO 2007/141736, 2007/145963, WO 2009/118375, WO 2010/055052 and WO 2011/036153. More particularly, the peptide aldehyde may be Cbz-RAY-H, Ac-GAY-H, Cbz-GAY-H, Cbz-GAL-H, Cbz-VAL-H, Cbz-GAF-H, Cbz-GAV-H, Cbz-GGY-H, Cbz-GGF-H, Cbz-RVY-H, Cbz-LVY-H, Ac-LGAY-H, Ac-FGAY-H, Ac-YGAY-H, Ac-FGAL-H, Ac-FGAF-H, Ac-FGVY-H, Ac-FGAM-H, Ac-WLVY-H, MeO-CO-VAL-H, MeNCO-VAL-H, MeO-CO-FGAL-H, MeO-CO-FGAF-H, MeSO₂-FGAL-H, MeSO₂-VAL-H, PhCH₂O(OH)(O)P-VAL-H, EtSO₂-FGAL-H, PhCH₂SO₂-VAL-H, PhCH₂O(OH)(O)P-LAL-H, PhCH₂O(OH)(O)P-FAL-H, or MeO(OH)(O)P-LGAL-H. Here, Cbz is benzyloxycarbonyl, Me is methyl, Et is ethyl, Ac is acetyl, H is hydrogen, and the other letters represent amino acid residues denoted by standard single letter notification (*e.g*., F = Phe, Y = Tyr, L = Leu).

Alternatively, the peptide aldehyde may have the formula as described in WO 2011/036153:

P-O-(Aᵢ-X')ₙ-Aₙ₊₁-Q

wherein Q is hydrogen, CH₃, CX"₃, CHX"₂, or CH₂X", wherein X" is a halogen atom;
wherein one X' is the "double N-capping group" CO, CO-CO, CS, CS-CS or CS-CO, most preferred urido (CO), and the other X' are nothing,
wherein n = 1-10, preferably 2-5, most preferably 2,
wherein each of Aᵢ and Aₙ₊₁ is an amino acid residue having the structure:
   - NH-CR"-CO- for a residue to the right of X' = -CO-, or
   - CO-CR"-NH- for a residue to the left of X' = -CO-
wherein R" is H- or an optionally substituted alkyl or alkylaryl group which may optionally include a hetero atom and may optionally be linked to the N atom, and
wherein P is hydrogen or any C-terminal protection group.

Examples of such peptide aldehydes include α-MAPI, β-MAPI, F-urea-RVY-H, F-urea-GGY-H, F-urea-GAF-H, F-urea-GAY-H, F-urea-GAL-H, F-urea-GA-Nva-H, F-urea-GA-Nle-H, Y-urea-RVY-H, Y-urea-GAY-H, F-CS-RVF-H, F-CS-RVY-H, F-CS-GAY-H, Antipain, GE20372A, GE20372B, Chymostatin A, Chymostatin B, and Chymostatin C. Further examples of peptide aldehydes are disclosed in WO 2010/055052 and WO 2009/118375, WO 94/04651, WO 98/13459, WO 98/13461, WO 98/13462, WO 2007/145963.

Alternatively to a peptide aldehyde, the protease inhibitor may be a hydrosulfite adduct having the formula X-B¹-NH-CHR-CHOH-SO₃M, wherein X, B¹ and R are defined as above, and M is H or an alkali metal, preferably Na or K.

The peptide aldehyde may be converted into a water-soluble hydrosulfite adduct by reaction with sodium bisulfite, as described in textbooks, *e.g*., March, J. Advanced Organic Chemistry, fourth edition, Wiley-Interscience, US 1992, p 895.

An aqueous solution of the bisulfite adduct may be prepared by reacting the corresponding peptide aldehyde with an aqueous solution of sodium bisulfite (sodium hydrogen sulfite, NaHSO₃); potassium bisulfite (KHSO₃) by known methods, *e.g*., as described in WO 98/47523; US 6,500,802; US 5,436,229; J. Am. Chem. Soc. (1978) 100, 1228; Org. Synth., Coll. vol. 7: 361.

The molar ratio of the above-mentioned peptide aldehydes (or hydrosulfite adducts) to the protease may be at least 1:1 or 1.5:1, and it may be less than 1000:1, more preferred less than 500:1, even more preferred from 100:1 to 2:1 or from 20:1 to 2:1, or most preferred, the molar ratio is from 10:1 to 2:1.

Formate salts (*e.g.*, sodium formate) and formic acid have also shown good effects as inhibitor of protease activity. Formate can be used synergistically with the above-mentioned protease inhibitors, as shown in WO 2013/004635. The formate salts may be present in the detergent composition in an amount of at least 0.1% w/w or 0.5% w/w, e.g., at least 1.0%, at least 1.2% or at least 1.5%. The amount of the salt is typically below 5% w/w, below 4% or below 3%.

In an embodiment, the protease is a metalloprotease and the inhibitor is a metalloprotease inhibitor, *e.g*., a protein hydrolysate based inhibitor (*e.g*., as described in WO 2008/134343).

### Adjunct materials

Any detergent components known in the art for use in laundry detergents may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in laundry detergents may be utilized. The choice of such ingredients is well within the skill of the artisan.

Dispersants - The detergent compositions of the present invention can also contain dispersants. In particular powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

Dye Transfer Inhibiting Agents - The detergent compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of *N*-vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

Fluorescent whitening agent - The detergent compositions of the present invention will preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino)stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

Soil release polymers - The detergent compositions of the present invention may also include one or more soil release polymers which aid the removal of soils from fabrics such as cotton and polyester based fabrics, in particular the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers are amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523. Furthermore random graft co-polymers are suitable soil release polymers. Suitable graft co-polymers are described in more detail in WO 2007/138054, WO 2006/108856 and WO 2006/113314. Other soil release polymers are substituted polysaccharide structures especially substituted cellulosic structures such as modified cellulose deriviatives such as those described in EP 1867808 or WO 2003/040279. Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

Anti-redeposition agents - The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

Rheology Modifiers are structurants or thickeners, as distinct from viscosity reducing agents. The rheology modifiers are selected from the group consisting of non-polymeric crystalline, hydroxy-functional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

### Bleaching Systems

The detergent compositions of the present invention may also include a bleaching system. Due to the incompatibility of the components there are still only few examples of liquid detergents combining bleach and enzymes (*e.g*., US 5,275,753 or WO 99/00478). The enzyme particles described in this invention can be used to separate bleach from enzyme in liquid detergents. The detergent may contain 0-50% of a bleaching system. Any bleaching system known in the art for use in laundry detergents may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO 98/17767. A particular family of bleach activators of interest was disclosed in EP624154 and particulary preferred in that family is acetyl triethyl citrate (ATC). ATC or a short chain triglyceride like triacetin has the advantage that it is environmental friendly as it eventually degrades into citric acid and alcohol. Furthermore acetyl triethyl citrate and triacetin has a good hydrolytical stability in the product upon storage and it is an efficient bleach activator. Finally ATC provides a good building capacity to the laundry additive. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae: 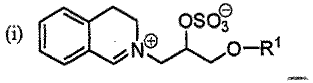 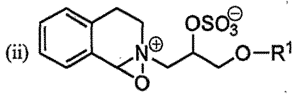 and mixtures thereof; wherein each R¹ is independently a branched alkyl group containing from 9 to 24 carbons or linear alkyl group containing from 11 to 24 carbons, preferably each R¹ is independently a branched alkyl group containing from 9 to 18 carbons or linear alkyl group containing from 11 to 18 carbons, more preferably each R¹ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, *e.g*., in WO 2007/087258, WO 2007/087244, WO 2007/087259 and WO 2007/087242. Suitable photobleaches may for example be sulfonated zinc phthalocyanine.

### Formulation of detergent products

The detergent composition of the invention may be in any convenient form, *e.g*., a bar, a homogenous tablet, a tablet having two or more layers, a pouch having one or more compartments, a regular or compact powder, a granule, a paste, a gel, or a regular, compact or concentrated liquid.

The detergent pouch of the present invention is configured as single or multi compartments (see *e.g*., WO 2009/098660 or WO 2010/141301). It can be of any form, shape and material which is suitable for holding the detergent composition, *e.g*., without allowing release of the composition from the pouch prior to water contact. The pouch is made from water-soluble film which encloses the inner volume (detergent composition). Said inner volume can be divided into compartments of the pouch. The water-soluble film is described above under "Water-soluble film". The pouch can comprise a solid laundry cleaning (detergent) composition or selected components thereof, and/or a liquid cleaning composition or selected components thereof, separated by the water-soluble film. The pouch may include compartments having any combination of solids and liquids, both in one or more separate compartments, and in shared compartments containing both solid and liquid ingredients. The pouch may include regions or compartments formed by different water-soluble films, which can be with or without enzymes. Accordingly, detergent ingredients can be separated physically from each other in different compartments, or in different layers of a tablet if the detergent is in that physical form. Thereby negative storage interaction between components can be avoided. Different dissolution profiles of each of the compartments can also give rise to delayed dissolution of selected components in the wash solution.

### Compositions, methods and uses

The present invention provides an enzyme composition comprising enzyme containing particles, wherein the particles comprise
i) at least one enzyme, preferably a detergent enzyme, and
ii) at least one polymer P,
where the polymer P is a hydrophobically modified polyvinyl alcohol, and the hydrophobic modifying groups are a mixture of keto-ester and butyryl groups such that the total degree of substitution (DS) is between about 3% and 20%.

In an embodiment, the polymer P is partially hydrolysed with high levels of hydrolysis in the range of about 60% to about 99%.

In another embodiment, the polymer P has a molecular weight in the range of about 1000 to 200000.

In another embodiment, the weight ratio of enzyme to polymer P is from 1:50 to 10:1.

In another embodiment, the volume average particle diameter of the enzyme containing particles is from 50 nm to 100 µm.

In another embodiment, the at least one enzyme and the at least one polymer P make up at last 50% of the enzyme containing particles.

In another embodiment, the enzyme is selected from the group consisting of protease, lipase, cutinase, an amylase, carbohydrase, cellulase, pectinase, mannanase, arabinase, galactanase, xylanase, DNAse, perhydrolase, oxidase, *e.g*., a laccase, and/or peroxidase. Preferably, the enzyme is a lipase.

In another embodiment, the enzyme composition is obtainable by spray drying a liquid composition containing the at least one enzyme and the at least one polymer P.

In an embodiment, the enzyme composition provides improved enzyme stability, as compared to an un-encapsulated enzyme; particularly in a liquid detergent composition.

In another aspect, the invention provides a method for preparing the enzyme composition described above, which method comprises drying a liquid composition containing the at least one enzyme and the at least one polymer P, as described above.

In yet another aspect, the invention provides for use of the enzyme composition, as described above, in a liquid laundry detergent composition.

In yet another aspect, the invention provides a liquid laundry detergent composition, which comprises at least one enzyme composition, as described above. Preferably, the enzyme composition comprises a lipase, and the detergent composition comprises a lipase sensitive detergent ingredient. Examples of lipase sensitive detergent ingredients are well-known to the skilled person and include, but are not limited to, ester based ingredients like perfume or hydrogenated castor oil.

In yet another aspect, the invention provides a detergent pouch comprising a compartment formed by a water-soluble film, and a detergent composition as described above.

The invention also provides for use of the compositions and methods above for improving the residual enzymatic activity after storage in the detergent of Table 5 for one week at 37°C, as compared to an un-encapsulated free enzyme (not comprised in the enzyme particle of the enzyme composition of the invention).

The present invention is further described by the following examples.

### EXAMPLES

Chemicals were commercial products of at least reagent grade. The lipase used in Example 1 has the amino acid sequence shown in SEQ ID NO: 1 (described in WO 2000/060063).

### EXAMPLE 1

### Polymer Synthesis

The synthesis of polymers A-H, as shown in Table 1, is described below.

**Table 1.**

| **Sample** | **Polymer** |
|---|---|
| A | Mowiol 4-98, KE: 5.11%, BU: 5% |
| B | Mowiol 10-98, KE: 7.00% |
| C | Mowiol 4-98, KE: 10.19%, BU: 5% |
| D | Mowiol 10-98, KE: 3.59%, BU: 5% |
| E | Mowiol 4-98, KE: 4.96% |
| F | Mowiol 10-98, KE: 9.87% |
| G | Mowiol 4-98, KE: 10.80% |
| H | Mowiol 10-98, KE: 8.62%, BU: 5% |

### The Synthesis of Keto-ester modified PVOH (polymers B, E, F and G)

Reagents were used according to the quantities shown in Table 2.

**Table 2.**

| **Sample** | **Polymer** | **Mowiol starting material grade** | **PVOH** | **Purified diketene acetone adduct** | **DMSO solvent** |
|---|---|---|---|---|---|
| B | Mowiol 10-98, KE: 7.00% | 10-98 | 60 g | 22.0 g | 540 g |
| E | Mowiol 4-98, KE: 4.96% | 4-98 | 60 g | 22.0 g | 540 g |
| F | Mowiol 10-98, KE: 9.87% | 10-98 | 60 g | 34.5 g | 540 g |
| G | Mowiol 4-98, KE: 10.80% | 4-98 | 60 g | 42.0 g | 540 g |

A 1 litre flanged flask was fitted with nitrogen inlet, internal thermocouple, overhead stirrer, condenser/bubbler and 2 subaseals (one for delivery of the adduct by syringe pump and second to take the internal thermocouple). The flask was purged with nitrogen and then charged with the Mowiol and the DMSO. The flask was then heated to 120°C (internal) by which time the Mowiol had dissolved. The acetone adduct was then added by syringe pump over a period of 1 hour and then allowed to react for a further 1 hour at 120°C. The condenser and bubbler were removed during the addition step to allow acetone to evaporate. The flask was then allowed to cool to room temperature and the polymer was precipitated into acetone. The polymer was then dried overnight at 50-60°C in a vacuum oven.

The polymer was then dissolved in water to give a 10% solution and then precipitated again into acetone. The polymer was then dried thoroughly in a vacuum oven at 50-60°C for a further 2 days before analysing by FTIR.

### The Synthesis of keto-ester and butyraldehyde modified PVOH (polymers A, C, D and H)

### Step 1 - Acetalisation

Reagents were used according to the quantities in Table 3.

**Table 3.**

| **Sample** | **Polymer** | **Mowiol grade** | **PVOH** | **Water** | **Butyr-aldehyde** | **2M HCl** |
|---|---|---|---|---|---|---|
| Precursor to A and C | Mowiol 4-98, BU: 5% | 4-98 | 134.25 g | 1208.25 g | 5.389 g | 11.2 ml |
| Precursor to D and H | Mowiol 10-98, BU: 5% | 10-98 | 147.67 g | 1329.03 g | 5.928 g | 12.3 ml |

PVOH and water were massed into a beaker and warmed to 80-90°C whilst being stirred using an overhead stirrer for 1-2 hours until all PVOH had dissolved.

Into a three-necked round bottom flask was massed the aqueous PVOH solution. The flask was then placed into an oil bath on a stirrer hotplate set at 60°C and fitted with a condenser (with bubbler) and a nitrogen feed line. The flask was then stoppered and purged with nitrogen for 5-10 minutes. After this time the concentrated HCI was added dropwise followed by the butyraldehyde. The reaction was then stirred under nitrogen for 4-5 hours at 60°C before being left to stir overnight at room temperature.

The reaction mixture was then poured into a jar and placed onto a stirrer plate. The pH of the solution was measured using pH dip strips and the solution was then neutralised by addition of ammonium hydroxide (whilst stirring). It was noted that, upon addition of ammonium hydroxide, some precipitation occurred. With time, this precipitate redissolved. The resulting solution was then precipitated into acetone. The product was then isolated by filtration under vacuum and was washed with acetone. The product was dried overnight in a vacuum oven at 40°C.

### Step 2 - Acetoacetylation

Reagents were used according to the quantities in Table 4.

**Table 4.**

| **Sample** | **Polymer** | **Starting material** | **Butyr-aldehyde modified PVOH** | **Purified diketene acetone adduct** | **DMSO solvent** |
|---|---|---|---|---|---|
| A | Mowiol 4-98, KE: 5.11%, BU: 5% | Precursor to A and C | 45 g | 12.42 g | 405 g |
| C | Mowiol 4-98, KE: 10.19%, BU: 5% | Precursor to A and C | 45 g | 25.0 g | 405 g |
| D | Mowiol 10-98, KE: 3.59%, BU: 5% | Precursor to D and H | 41 g | 11.7 g | 769 g |
| H | Mowiol 10-98, KE: 8.62%, BU: 5% | Precursor to D and H | 39 g | 22.3 g | 741 g |

A 1 litre flanged flask was fitted with nitrogen inlet, internal thermocouple, overhead stirrer, condenser/bubbler and 2 subaseals (one for delivery of the adduct by syringe pump and second to take the internal thermocouple). The flask was purged with nitrogen and then charged with the Mowiol and the DMSO. The flask was then heated to 120°C (internal) by which time the Mowiol had dissolved. The acetone adduct was then added by syringe pump over a period of 1 hour and then allowed to react for a further 1 hour at 120°C. The condenser and bubbler were removed during the addition step to allow acetone to evaporate. The flask was then allowed to cool to room temperature and the polymer was precipitated into acetone. The polymer was then dried overnight at 50-60°C in a vacuum oven.

The polymer was then dissolved in water to give a 10% solution and then precipitated again into acetone. The polymer was then dried thoroughly in a vacuum oven at 50-60°C for a further 2 days before analysing by FTIR.

### Preparation of Liquid Laundry Detergent

Liquid laundry model detergent was prepared from the following compositions (all percentages in w/w):

**Table 5.**

| **Component** | **Amount** |
|---|---|
| (C₁₀-C₁₃) alkylbenzene-sulfonic acid (LAS) | 12% |
| Nonionic surfactant, alcohol ethoxylate, (C13AE8EO) | 9% |
| Soy Fatty acid (Edenor SJ) | 6% |
| Coco fatty acid (Radiacid 0631) | 5% |
| Triethanolamine | 2% |
| Sodium citrate dihydrat | 1% |
| Phosphonate (Dequest 2066 C2) | 3% |
| Propane-1,2-diol | 5% |
| Ethanol | 4.5% |
| Propan-2-ol | 0.5% |
| pH (adjusted with NaOH) | 9.2 |
| De-ionized water | Ad 100% |

### Preparation of water phase

6.75 g of polymer solution (20%) was mixed with 0.75 g of an aqueous solution of lipase (120 mg active enzyme per gram).

### Preparation of oil phase

295 g Whiteway 15 mineral oil (technical white oil from Statoil) was mixed with 1.5 ml Sorbitan Sesquioleate (Sigma) and 1.5 ml of a 20% solution of high-MW hydrolyzed copolymer of styrene, stearyl methacrylate and maleic anhydride terpolymer emulsifier in mineral oil(see WO 99/01534, Example 5).

### Preparation of particles in the emulsion

The oil phase was heated to between 30-40°C and mixed with a Silverson L4RT high shear mixer for 1 minute at 1600 rpm. Water phase was slowly added to the oil phase under continued mixing. After addition of the water phase the mixing was continued for 3 min and the resulting emulsion transferred to a beaker. The emulsion was dried under vacuum at 30-40°C for 3-6 hours while gently stirred by a dog-bone magnet to avoid agglomeration.

### Isolation of matrix particle slurry

The resulting dispersion was centrifuged at 3000 RPM for 3 min in a Heraus Sepatech Labofuge A with a head diameter of 25 cm to settle the particles. The supernatant oil phase was decanted off and discarded and the particles are re-suspended in a minimal amount of Whiteway oil (about 10 ml).

The particle size (diameter) of the resulting encapsulated enzyme was in the span of 1-30 microns.

### Testing stability in detergent

0.25-1% capsule slurry was added to the liquid laundry detergent and the detergent was incubated in closed glasses for 1 week at 37°C and for some samples also 8 weeks at 30°C. A detergent reference was produced using the un-encapsulated enzyme concentrate added directly to the detergent to a similar enzyme activity level.

After storage, the activity was measured by using standard enzyme analytical methods (hydrolysis of p-nitrophenyl palmitate at 37°C, pH 8.0) after an initial 1:100 dilution in demineralized water to facilitate release of enzyme from the capsules. Residual activities were calculated relative to the samples stored at -18°C.

### Results

**Table 6.**

| **Sample** | **Polymer** | **Residual enzyme activity** | |
|---|---|---|---|
| | | **1w@37°C** | **8w@30°C** |
| A | Mowiol 4-98, KE: 5.11%, BU: 5% | 65% | 76% |
| B | Mowiol 10-98, KE: 7.00% | 59% | 67% |
| C | Mowiol 4-98, KE: 10.9%, BU: 5% | 56% | 72% |
| D | Mowiol 10-98, KE: 3.59%, BU: 5% | 53% | not analysed |
| E | Mowiol 4-98, KE: 4.96% | 52% | not analysed |
| F | Mowiol 10-98, KE: 9.87% | 50% | not analysed |
| G | Mowiol 4-98, KE: 10.80% | 50% | not analysed |
| H | Mowiol 10-98, KE: 8.62%, BU: 5% | 48% | not analysed |
| I | Reference, un-encapsulated | 43% | 54% |

The results in Table 6 show that the residual activity of the lipase is improved by encapsulating the lipase in the polymer matrix (to form an enzyme particle).

## Claims

1. Enzyme composition comprising enzyme containing particles, wherein the particles comprise
i) at least one enzyme, and
ii) at least one polymer P,
where the polymer P is a hydrophobically modified polyvinyl alcohol, and the hydrophobic modifying groups are a mixture of keto-ester and butyryl groups such that the total degree of substitution (DS) is between about 3% and 20%.

2. The composition of claim 1, wherein the polymer P is partially hydrolysed with high levels of hydrolysis in the range of about 60% to about 99%.

3. The composition of claim 1 or 2, wherein the polymer P has a molecular weight in the range of about 1000 to 200000.

4. The composition of any one of claims 1 to 3, wherein the weight ratio of enzyme to polymer P is from 1:50 to 10:1.

5. The composition of any one of claims 1 to 4, wherein volume average particle diameter of the enzyme containing particles is from 50 nm to 100 µm.

6. The composition of any one of claims 1 to 5, wherein the at least one enzyme and the at least one polymer P make up at last 50% of the enzyme containing particles.

7. The composition of any one of claims 1 to 6, wherein the enzyme is a lipase.

8. The composition of any one of claims 1 to 7, which is obtainable by spray drying a liquid composition containing the at least one enzyme and the at least one polymer P.

9. A method for preparing a composition according to any one of claims 1 to 8, which method comprises drying a liquid composition containing the at least one enzyme and the at least one polymer P.

10. Use of the enzyme composition of any one of claims 1 to 8 in a liquid laundry detergent composition.

11. A liquid laundry detergent composition, which comprises at least one enzyme composition according to any one of claims 1 to 8.

12. The detergent composition of claim 11, comprising an enzyme composition according to claim 9, and a lipase sensitive detergent ingredient.

13. A detergent pouch comprising a compartment formed by a water-soluble film, and a detergent composition according to claim 11 or 12.

## Patentansprüche

1. Enzymzusammensetzung, die enzymhaltige Partikel umfasst, wobei die Partikel
i) mindestens ein Enzym, und
ii) mindestens ein Polymer P umfassen,
wobei das Polymer P ein hydrophob modifizierter Polyvinylalkohol ist, und die hydrophoben modifizierenden Gruppen ein Gemisch aus Ketoester- und Butyrylgruppen sind, so dass der Gesamtsubstitutionsgrad (DS) zwischen etwa 3% und 20% liegt.

2. Zusammensetzung nach Anspruch 1, wobei das Polymer P mit hohen Spiegeln an Hydrolyse im Bereich von etwa 60% bis etwa 99% teilweise hydrolysiert ist.

3. Zusammensetzung nach Anspruch 1 oder 2, wobei das Polymer P ein Molekulargewicht im Bereich von etwa 1000 bis 200 000 aufweist.

4. Zusammensetzung nach einem beliebigen der Ansprüche 1 bis 3, wobei das Gewichtsverhältnis von Enzym zu Polymer P von 1:50 bis 10:1 beträgt.

5. Zusammensetzung nach einem beliebigen der Ansprüche 1 bis 4, wobei der volumengemittelte Partikeldurchmesser der enzymhaltigen Partikel von 50 nm bis 100 µm beträgt.

6. Zusammensetzung nach einem beliebigen der Ansprüche 1 bis 5, wobei das mindestens eine Enzym und das mindestens eine Polymer P mindestens 50% der enzymhaltigen Partikel ausmachen.

7. Zusammensetzung nach einem beliebigen der Ansprüche 1 bis 6, wobei das Enzym eine Lipase ist.

8. Zusammensetzung nach einem beliebigen der Ansprüche 1 bis 7, die durch Sprühtrocknen einer flüssigen Zusammensetzung erhältlich ist, die das mindestens eine Enzym und das mindestens eine Polymer P enthält.

9. Verfahren zum Herstellen einer Zusammensetzung gemäß einem beliebigen der Ansprüche 1 bis 8, wobei das Verfahren das Trocknen einer flüssigen Zusammensetzung umfasst, die das mindestens eine Enzym und das mindestens eine Polymer P enthält.

10. Verwendung der Enzymzusammensetzung gemäß einem beliebigen der Ansprüche 1 bis 8 in einer flüssigen Waschmitteldetergenszusammensetzung.

11. Flüssige Waschmitteldetergenszusammensetzung, die mindestens eine Enzymzusammensetzung gemäß einem beliebigen der Ansprüche 1 bis 8 umfasst.

12. Detergenszusammensetzung nach Anspruch 11, die eine Enzymzusammensetzung gemäß Anspruch 9 und einen lipaseempfindlichen Detergensbestandteil umfasst.

13. Detergensbeutel, der eine Kammer, die durch einen wasserlöslichen Film gebildet ist, und eine Detergenszusammensetzung gemäß Anspruch 11 oder 12 umfasst.

## Revendications

1. Composition enzymatique comprenant des particules contenant une enzyme, dans laquelle les particules comprennent
i) au moins une enzyme, et
ii) au moins un polymère P,
dans laquelle le polymère P est un alcool polyvinylique modifié hydrophobiquement, et les groupes modificateurs hydrophobe sont un mélange de groupes céto-ester et butyryle tel que le degré total de substitution (DS) soit compris entre environ 3 % et 20 %.

2. Composition selon la revendication 1, dans laquelle le polymère P est partiellement hydrolysé avec des niveaux élevés d'hydrolyse situés dans la plage allant d'environ 60 % à environ 99 %.

3. Composition selon la revendication 1 ou 2, dans laquelle le polymère P a une masse moléculaire située dans la plage allant d'environ 1 000 à 200 000.

4. Composition selon l'une quelconque des revendications 1 à 3, dans laquelle le rapport en poids de l'enzyme au polymère P est de 1/50 à 10/1.

5. Composition selon l'une quelconque des revendications 1 à 4, dans laquelle la granulométrie moyenne en volume des particules contenant une enzyme est de 50 nm à 100 µm.

6. Composition selon l'une quelconque des revendications 1 à 5, dans laquelle ladite au moins une enzyme et ledit au moins un polymère P constituent au moins 50 % des particules contenant une enzyme.

7. Composition selon l'une quelconque des revendications 1 à 6, dans laquelle l'enzyme est une lipase.

8. Composition selon l'une quelconque des revendications 1 à 7, qui peut être obtenue par séchage par pulvérisation d'une composition liquide contenant ladite au moins une enzyme et ledit au moins un polymère P.

9. Méthode pour préparer une composition selon l'une quelconque des revendications 1 à 8, laquelle méthode comprend le séchage d'une composition liquide contenant ladite au moins une enzyme et ledit au moins un polymère P.

10. Utilisation de la composition enzymatique de l'une quelconque des revendications 1 à 8 dans une composition détergente liquide pour le linge.

11. Composition détergente liquide pour le linge, qui comprend au moins une composition enzymatique selon l'une quelconque des revendications 1 à 8.

12. Composition détergente selon la revendication 11, comprenant une composition enzymatique selon la revendication 9, et un ingrédient détergent sensible aux lipases.

13. Sachet de détergent comprenant un compartiment formé par un film soluble dans l'eau, et une composition détergente selon la revendication 11 ou 12.


### SEQUENCE LISTING

<110> Novozymes A/S
<120> Detergents and compositions with enzymatic polymer particles
<130> 12826-WO-PCT
<160> 1
<170> PatentIn version 3.5
<210> 1
   <211> 269
   <212> PRT
   <213> Artificial
<220>
   <223> Thermomyces lanuginosus lipase variant
<400> 1
   Glu Val Ser Gln Asp Leu Phe Asn Gln Phe Asn Leu Phe Ala Gln Tyr
   1               5                   10                  15
   Ser Ala Ala Ala Tyr Cys Gly Lys Asn Asn Asp Ala Pro Ala Gly Thr
               20                  25                  30
   Asn Ile Thr Cys Thr Gly Asn Ala Cys Pro Glu Val Glu Lys Ala Asp
           35                  40                  45
   Ala Thr Phe Leu Tyr Ser Phe Glu Asp Ser Gly Val Gly Asp Val Thr
       50                  55                  60
   Gly Phe Leu Ala Leu Asp Asn Thr Asn Lys Leu Ile Val Leu Ser Phe
   65                  70                  75                  80
   Arg Gly Ser Arg Ser Ile Glu Asn Trp Ile Gly Asn Leu Asn Phe Asp
                   85                  90                  95
   Leu Lys Glu Ile Asn Asp Ile Cys Ser Gly Cys Arg Gly His Asp Gly
               100                 105                 110
   Phe Thr Ser Ser Trp Arg Ser Val Ala Asp Thr Leu Arg Gln Lys Val
           115                 120                 125
   Glu Asp Ala Val Arg Glu His Pro Asp Tyr Arg Val Val Phe Thr Gly
       130                 135                 140
   His Ser Leu Gly Gly Ala Leu Ala Thr Val Ala Gly Ala Asp Leu Arg
   145                 150                 155                 160
   Gly Asn Gly Tyr Asp Ile Asp Val Phe Ser Tyr Gly Ala Pro Arg Val
                       165                 170                 175
   Gly Asn Arg Ala Phe Ala Glu Phe Leu Thr Val Gln Thr Gly Gly Thr
               180                 185                 190
   Leu Tyr Arg Ile Thr His Thr Asn Asp Ile Val Pro Arg Leu Pro Pro
           195                 200                 205
   Arg Glu Phe Gly Tyr Ser His Ser Ser Pro Glu Tyr Trp Ile Lys Ser
       210                 215                 220
   Gly Thr Leu Val Pro Val Arg Arg Arg Asp Ile Val Lys Ile Glu Gly
   225                 230                 235                 240
   Ile Asp Ala Thr Gly Gly Asn Asn Gln Pro Asn Ile Pro Asp Ile Pro
                   245                 250                 255
   Ala His Leu Trp Tyr Phe Gly Leu Ile Gly Thr Cys Leu
               260                 265